# EUROPÄISCHE PATENTSCHRIFT

(11) **EP 0 682 925 B1**
(45) Veröffentlichungstag und Bekanntmachung des Hinweises auf die Patenterteilung: **12.09.2001**
(21) Anmeldenummer: 95109878.9
(22) Anmeldetag: 16.06.1992
(51) Int. Cl.: A61F 2/38

(54) **Gelenkprothese, insbesondere Kniegelenkprothese**
Joint prosthesis, particularly for the knee
Prothèse d'articulation, en particulier du genou

(30) Priorität: 17.06.1991 CH 179591; 17.06.1991 CH 179691
(43) Veröffentlichungstag der Anmeldung: 22.11.1995
(62) Teilanmeldung aus: 92810464.5
(73) Patentinhaber: Bähler, André, CH-8032 Zürich (CH)
(72) Erfinder: Bähler, André, CH-8032 Zürich (CH)
(74) Vertreter: Riederer, Conrad A., Dr.

(56) Entgegenhaltungen:
- WO-A-92/08424
- DE-U- 9 110 504
- FR-A- 2 290 883
- FR-A- 2 663 536
- GB-A- 2 061 730
- US-A- 4 207 627

## Beschreibung

Die Erfindung betrifft eine Gelenkprothese, insbesondere Kniegelenkprothese, mit mindestens einem ersten Prothesenteil, das einen Verankerungsabschnitt und mindestens einen Drehgelenkabschnitt aufweist, einem zweiten Prothesenteil, das einen Verankerungsabschnitt aufweist, und mindestens einen Meniskusteil mit mindestens einem Führungsorgan, welches von einer Führungsbahn in angenähert physiologischer Gleitrichtung geführt wird, um eine Translationsbewegung zu ermöglichen.

Eine Gelenkprothese gemäß der Merkmalen des Oberbegriffs des Anspruchs 1 ist aus der GB-A-2 061 730 bekannt.

Ein bis heute nur unvollkommen gelöstes Problem der Endoprothetik ist die unveränderte Aufrechterhaltung eines stabilen Knochen/Implantat-Verbundes über lange Jahre, im Idealfall bis zum Tode des Prothesenträgers. Es sind vor allem zwei Faktoren, welche dagegen wirken. Es sind dies zum einen die an der Knochen/Implantat-Grenze, dem sogenannten Interface, auftretenden, nach Betrag und Richtung wechselnden Kräfte, insbesondere Scherkräfte, und zum anderen knochenzerstörende, biologische Gewebereaktionen auf Fremdkörpermaterial, insbesondere auf Abriebpartikel von den Prothesenteilen. Soll also die Langlebigkeit einer Gelenkprothese erhöht werden, so sind durch geeignete Massnahmen die am Interface angreifenden Wechselkräfte klein zu halten und der an den Gleitflächen auftretende Verschleiss zu minimieren. Dies kann auf verschiedene Weise geschehen, doch sind nicht alle Methoden gleichermassen gangbar. Es genügt nämlich nicht, nur die genannten technischen Aspekte zu berücksichtigen, sondern es muss auch auf die anatomischen und physiologischen Verhältnisse Rücksicht genommen werden. Insbesondere bei Gelenken mit einer verhältnismässig komplizierten Kinematik, wie z.B. beim Kniegelenk, kann es zu schwer zu lösenden Zielkonflikten kommen.

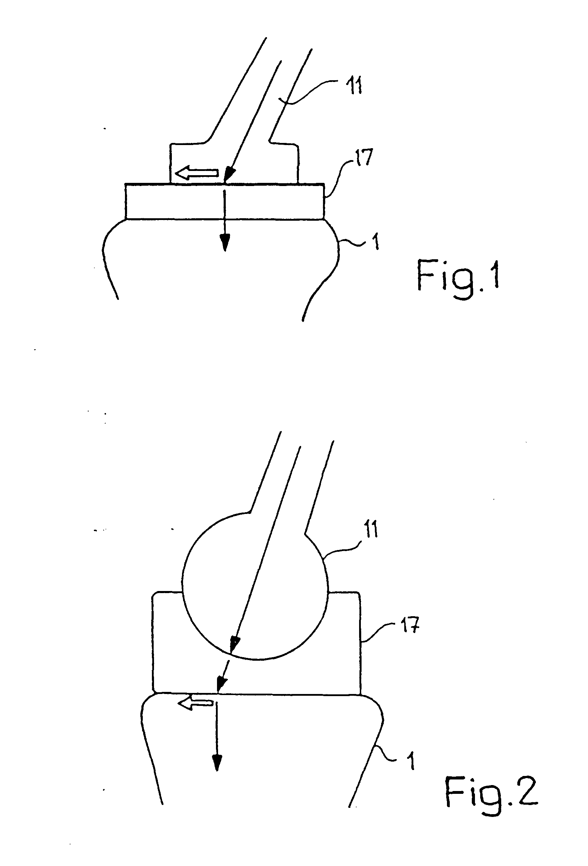

Bekanntlich ist bei Gleitlagern der Verschleiss umso geringer, je kleiner der Flächendruck der aufeinander reibenden Gleitpartner ist. Der Flächendruck, und damit auch der resultierende Verschleiss, ist klein, wenn die eigentliche Kontaktfläche der beiden Gleitpartner gross ist. Die Kontaktfläche ist dann gross, wenn die Gleitflächen möglichst gross und kongruent ausgebildet sind. Typische Beispiele hierfür sind das Schlittengelenk (Fig. 1) und das Scharniergelenk (Fig. 2). Obwohl das Schlittengelenk im Prinzip als ein Scharniergelenk mit unendlichem Radius betrachtet werden kann, bestehen aber ausser dem gemeinsamen Merkmal des minimierten Verschleisses grundsätzliche Unterschiede. Das Schlittengelenk ist translativ frei beweglich, besitzt aber keine Drehachse. Umgekehrt besitzt das Scharniergelenk eine Drehachse, ist aber translativ gefangen. Dies hat unterschiedliches Verhalten gegenüber von aussen einwirkenden Kräften zur Folge.

Beim Schlittengelenk bewirkt z.B. eine schräg von oben kommende Kraft eine Verschiebung des Gelenkkopfes auf der Gleitfläche in Richtung der horizontalen Komponente, ohne dass der untere Gelenkteil und damit dessen Interface von dieser Horizontalkraft selbst betroffen würde.

Beim Scharniergelenk hingegen geht die gleiche Kraft durch das Gelenk hindurch, ohne im Gelenk eine Bewegung auszulösen, induziert jedoch am Interface des unteren Gelenkteils eine Horizontalkomponente und damit eine unerwünschte Scherkraft, die zudem ständig ihre Richtung wechselt, wenn der obere Gelenkteil hin und her pendelt. Vereinfacht gesagt wird mit dem Scharniergelenk der Vorteil der Drehbeweglichkeit mit dem Nachteil der translativen Immobilität und der Scherkraftbelastung am Interface erkauft. Nun sind Körpergelenke selten reine Scharnier- oder Schlittengelenke, vielmehr findet sich, wie z.B. beim Kniegelenk, eine Kombination davon, indem der Drehbewegung eine gleichzeitige Translation überlagert ist.

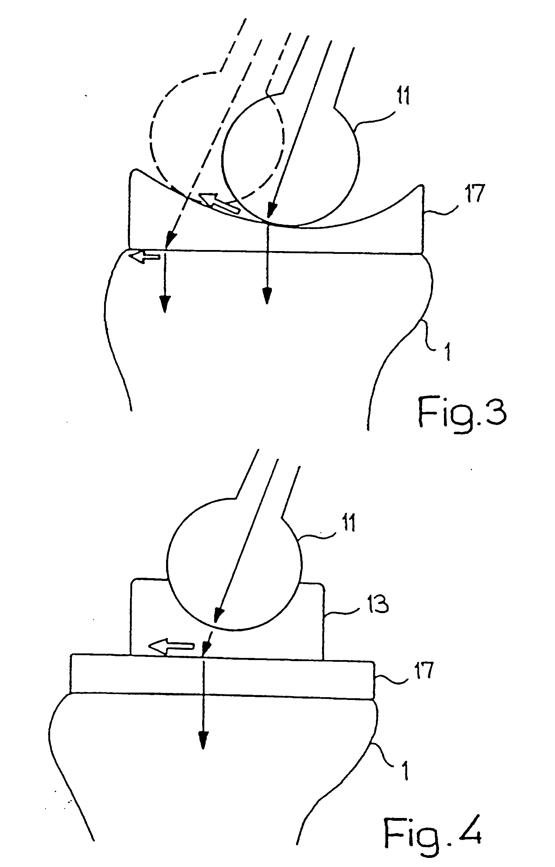

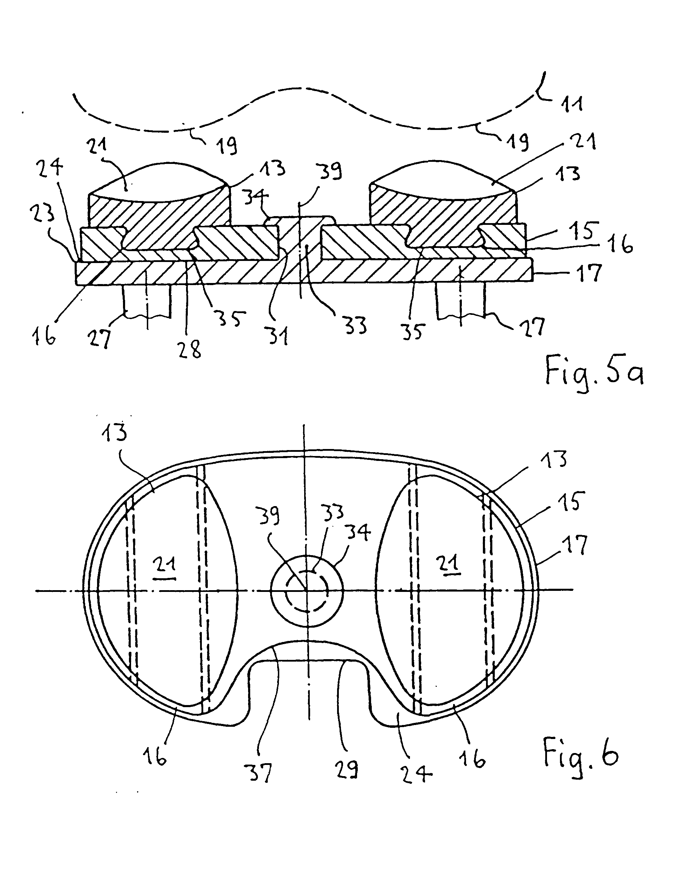

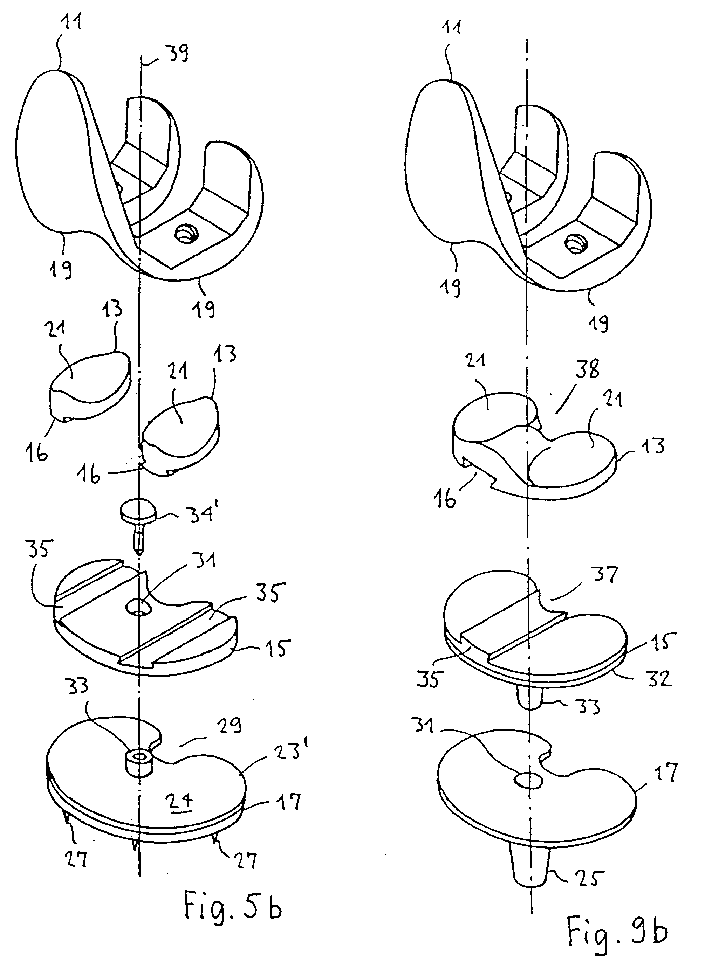

Um diese Kombination von Bewegungen zu ermöglichen, muss das Scharniergelenk "geöffnet" werden, d.h. die Kongruenz der Gleitflächen muss herabgesetzt werden (Fig. 3). Dies bedeutet aber, kleine Kontaktflächen mit entsprechend grossem Flächendruck, was erhöhten Verschleiss zur Folge hat. Der Verschleiss wird zusätzlich durch die repetitive Translation des Gelenkkopfes auf der Gleitfläche erhöht, was infolge des fokussierten Flächendrucks zu einem Walkprozess mit besonders schneller Materialermüdung des untenliegenden Gelenkteils führt. Zudem schützt die Inkongruenz nicht unbedingt vor Scherkräften am Interface. Ueberhöhte Gleitflächen an der Peripherie wirken sich kräftemässig analog aus, wie ein Scharniergelenk. Diese Nachteile, erhöhter Verschleiss und Scherkräfte am Interface mit den damit verbundenen negativen Auswirkungen, sind bei allen Prothesen dieser Bauart wohlbekannt.

Eine andere bekannte Lösung ist die Kombination von Schlitten- und Scharniergelenk auf zwei Ebenen mit Hilfe eines Meniskusteils (Fig. 4). Der Drehbewegung des Scharniergelenks wird gewissermassen die Translationsbewegung des Schlittengelenks unterlagert. Bei diesem Prinzip bleibt die Kongruenz der Gelenkflächen voll erhalten, und der Verschleiss wird minimiert. Zudem werden die durch das Scharniergelenk durchgehenden Kräfte nicht auf das Interface übertragen, sondern im Meniskusteil in Translationsbewegung umgesetzt. Es existieren auch Prothesen dieser Bauart und sind als sogenannte Meniskusknie bekannt.

So werden in der EP-B-0 021 421 zwei verschiedene Kniegelenkprothesen beschrieben, welche auch als "Oxford-Knie" und "New Jersey-Knie" bezeichnet werden. Das Oxford-Knie weist zwei Femurteile, zwei Meniskusteile und zwei Tibiateile auf. Die Femurteile bestehen aus je einem sphärischen Segment, das einen Verankerungsabschnitt zur Verankerung im Femur aufweist. Die Meniskusteile sind runde Scheiben mit einer sphärischen Vertiefung zur Lagerung des entsprechenden Femurteils. Die Tibiateile besitzen einen Verankerungsabschnitt zur Verankerung in der Tibia und ein Plateau, auf welchem das Meniskusteil gleiten kann. Als nachteilig erweist sich dabei, dass bei einer Flexion von 90 Grad und mehr das Meniskusteil über das Plateau teilweise hinausgeschoben und eventuell ganz disloziert werden kann. Die gleiche Gefahr besteht auch, wenn aus irgendwelchen Gründen die Spannung der nach der Operation verbliebenen Gelenkbänder nachlässt und sich somit die Femurteile von den Meniskusteilen abheben lassen. In solchen Fällen ist stets eine erneute Operation des Kniegelenks notwendig.

Das New Jersey-Knie begegnet der beschriebenen Gefahr des Hinausschiebens durch spezielle Vorrichtungen. Am Tibiateil, das für beide Meniskusteile vorgesehen ist, befinden sich zwei schwalbenschwanzartige, bogenförmige Nuten, durch welche die Zwischenteile zwangsgeführt werden. Die Bogen sind jeweils gegen das Zentrum zu gerichtet, so dass das Meniskusteil beim Beugen des Knies nicht unkontrolliert rückwärtsgleiten und über das Plateau hinausgeschoben werden kann, sondern am dort noch verbliebenen zentralen Knochenzapfen anschlägt. Die Schwalbenschwanzform andererseits verhindert ein Luxieren des Zwischenteils beim Nachlassen der Bänderspannung. Der Nachteil dieser Ausführung ist die Zwangsführung der Meniskusteile auf eine vorbestimmte Bahn, welche nur für eine einzige, singuläre Gelenkstellung eine Kongruenz der Gelenkflächen zwischen Femurteil und Meniskusteil zulässt, für jede andere Gelenkstellung jedoch zu einer verschleissfördernden Inkongruenz der Gelenkflächen führt. Eine Inkongruenz entsteht, weil die am Femur fest verankerten Femurteile stets im gleichen Abstand zueinander stehen und eine gemeinsame Drehachse haben, die Meniskusteile jedoch beim Vor- und Rückwärtsgleiten auf der bogenförmigen Bahn sich seitlich einander nähern oder voneinander entfernen, wobei ausserdem die Scharniergelenkachsen stetig ihre Stellung zueinander ändern. Dasselbe, nur im umgekehrten Sinne, geschieht auch bei Rotationsbewegungen um eine Achse senkrecht zum Plateau. Der ständig wechselnde Grad dieser Prothese innewohnenden Inkongruenz der Scharniergelenkflächen kompromittiert natürlich das verschleissminimierende Prinzip des Meniskusknie in grundsätzlicher Weise.

In der EP-B-0 018 364 wird eine Kniegelenkprothese beschrieben, auch "rotating platform" genannt, welche ein Femurteil, ein Tibiateil und ein Meniskusteil aufweist. Das Femurteil besitzt einen Verankerungsabschnitt zur Befestigung im Femur und zwei Kondylen. Das Tibiateil besitzt einen Verankerungsabschnitt zur Befestigung in der Tibia und eine zentrale Bohrung zur Aufnahme eines Drehzapfens des Meniskusteils. Das Meniskusteil besitzt einen in die zentrale Bohrung des Tibiateils passenden Drehzapfen sowie zwei konkave Gleitlager zur Lagerung der Kondylen des Femurteils. Dadurch weist diese Prothese den Vorteil der verschleissmindernden Kongruenz der Gleitflächen des Scharniergelenks auf und zudem wird die Luxation des Meniskusteils wegen des Drehzapfens verhindert.

Im Gegensatz jedoch zu den vorhergehend diskutierten Beispielen besteht wegen des Drehzapfens keine Möglichkeit der translativen Bewegung des Femurteils relativ zum Tibiateil, sondern nur die Möglichkeit der Rotation um die Achse des Drehzapfens. Es handelt sich hier also um ein klassisches Scharniergelenk mit der zusätzlichen Freiheit der Rotation um eine senkrecht zu diesem Gelenk stehende Achse. Demzufolge gehen auch schräg von oben kommende Kräfte, wie sie z.B. durch den Muskelzug oder die Gewichtsbelastung beim Aufsetzen des Fusses entstehen, durch das Gelenk hindurch auf das Interface und induzieren dort die unerwünschten Scherkräfte. Auch in diesem Fall wird das Prinzip des Meniskusknie kompromittiert, indem die verschleissmindernde Kongruenz der Gelenkflächen mit dem Verlust der verankerungsschonenden Translationsmöglichkeit und der damit verbundenen natürlichen Kniekinematik erkauft wird.

Die WO-A-9208424, welche eine ältere nachveröffentlichte Anmeldung darstellt, zeigt eine Kniegelenkprothese mit einem ersten und einem zweiten Prothesenteil und einem dazwischen liegenden Meniskus teil, welches ein Langloch aufweist. In diesem Langloch befindet sich ein kurzes Führungsstück, das mit einer im zweiten Prothesenteil eingeschraubten Ansatzschraube drehbar befestigt ist. Dies ermöglicht dem Meniskusteil sowohl eine Rotationsbewegung als auch eine Anterior-Posterior-Bewegung. Die Rotationsbewegung wird durch Anschläge begrenzt. Die Anterior-Posterior-Bewegung, also Translationsbewegung, wird durch die Länge des Langlochs begrenzt. Nachteilig bei dieser Prothese ist die relativ kurze Translationsbewegung, weil wegen Platzmangel das Langloch notgedrungen kurz gehalten werden muss. Das kurze Langloch bedingt relativ kleine Führungsflächen am Führungs-stück und dementsprechend hohe spezifische Flächenpressungen. Dies wiederum führt zu hohem Verschleiss, wobei auch ein Verkanten und Verklemmen möglich ist. Als nachteilig erweist sich aber auch das ständige Anschlagen des Meniskusteils am Ende jeder Translationsbewegung, und die dadurch an der Implantat/Knochen-Grenze auftretenden wechselnden Kräfte, wie dies bereits einleitend geschildert wurde.

Es ist daher Aufgabe der vorliegenden Erfindung, die beschriebenen Nachteile bekannter Prothesen zu vermeiden. Insbesondere soll eine Prothese geschaffen werden, welche nicht nur Gelenkflächenkongruenz und Translationsmöglichkeit des sogenannten Meniskusknies aufweist, sondern diese auch bei Rotationsbewegungen aufrecht erhält und weitgehende Luxationssicherheit des Meniskusteils gewährleistet sowie einen annähernd normalen, physiologischen Bewegungsablauf ermöglicht.

Diese Aufgabe wird gemäss der Erfindung mit einer Gelenkprothese nach Anspruch 1 gelöst.

Dies ermöglicht einen Bewegungsablauf, der weitgehend dem normalen physiologischen Bewegungsablauf entspricht. So verlagern sich bei der Flexion die Kondylen des ersten Prothesenteils, also bei einer Kniegelenkprothese jene des Femurteils, von anterior nach posterior. Gleichzeitig kann aber auch eine Drehbewegung um eine Achse senkrecht zum Plateau des zweiten Prothesenteils stattfinden. Sowohl bei der Flexion als auch bei der Extension sind daher ähnliche Verhältnisse gegeben wie beim natürlichen Knie. Auch ist in jeder Gelenkstellung die Kongruenz der Scharniergelenkflächen von Femurteil und Meniskusteil gewährleistet, da die auf dem Meniskusteil angeordneten Gelenkflächen zueinander unbeweglich sind. Von oben kommende Kräfte erzeugen keine Scherkräfte am Interface, sondern werden durch die Meniskusteile in Translationsbewegung umgesetzt. Schliesslich ist auch die Gefahr der gefürchteten Dislokation des Zwischenteils oder der Meniskusteile bei der Flexion gebannt.

Die jeweilige Führungsbahn kann geradlinig verlaufen. Dies ermöglicht eine einfache Herstellung der Prothese.

Bei einem vorteilhaften Ausführungsbeispiel der Erfindung ist am zweiten Prothesenteil ein Drehzapfen ausgebildet, um welchen das Zwischenteil verschwenkbar gelagert ist. Dies ergibt eine besonders einfache Konstruktion.

Es ist auch möglich, statt am zweiten Prothesenteil am Zwischenteil einen Drehzapfen auszubilden, welcher im zweiten Prothesenteil verschwenkbar gelagert ist. In diesem Fall kann der Drehzapfen relativ lang ausgebildet werden, so dass keine besonderen Sicherungsmassnahmen getroffen werden müssen, um ein Abheben aus dem zweiten Prothesenteil zu verhindern.

Zweckmässigerweise besitzt das zweite Prothesenteil eine Gleitfläche, auf welcher das Zwischenteil gelagert ist. Diese Gleitfläche kann verhältnismässig gross gehalten werden, so dass die Flächenpressung klein bleibt und eine Abnützung praktisch vermieden wird.

Vorteilhaft sind Mittel vorgesehen, um ein Abheben des Zwischenteils vom zweiten Prothesenteil zu verhindern. Weiter ist die Konstruktion vorzugsweise so gestaltet, dass die Beweglichkeit des Meniskusteils relativ zum Zwischenteil auf Bewegungen entlang der Führungsbahn eingeschränkt ist. So ist es möglich, dass Führungsbahn und Führungsteil als Nut und Steg, als Schwalbenschwanz, als Ueberdachung oder bogenförmige Umfassung ausgebildet sind. Durch diese Ausgestaltung wird verhindert, dass das Führungsteil die Führungsbahn verlässt, wenn aus irgendwelchen Gründen die Spannung der nach der Operation verbliebenen Gelenkbänder nachlässt.

Die Drehgelenkabschnitte können verschiedenartig ausgebildet sein, um eine Drehbewegung zu ermöglichen. Eine vorteilhafte Ausführungsform sieht vor, dass der Drehgelenkabschnitt des ersten Prothesenteils durch zwei Kondylen gebildet ist, und für jede dieser Kondylen ein Meniskusteil mit einer Lagerfläche für die Kondyle vorgesehen ist. Dies ergibt eine Prothese, die dem natürlichen Gelenkaufbau, wie er beispielsweise beim Kniegelenk zu finden ist, sehr nahe kommt.

Eine andere vorteilhafte Ausführungsform sieht vor, dass der Drehgelenkabschnitt des ersten Prothesenteils durch zwei Kondylen gebildet ist und dass ein für beide Kondylen gemeinsames Meniskusteil mit einer Lagerfläche für jeden der Kondylen vorgesehen ist. Auch diese Gestaltung ermöglicht einen Bewegungsablauf, der dem natürlichen physiologischen Bewegungsablauf sehr nahekommt. Es genügt dabei, für den gemeinsamen Meniskusteil eine einzige Führungsbahn vorzusehen. Es ist aber auch möglich, dass das gemeinsame Meniskusteil durch zwei in einem Abstand voneinander angeordnete Führungsbahnen geführt wird. Dies trägt dazu bei, eine Abnützung praktisch zu verhindern.

Möglich ist auch eine monokompartimentäre Ausführung der Gelenkprothese. So kann der Drehgelenkabschnitt des ersten Prothesenteils durch eine Kondyle gebildet sein und das Zwischenteil ein Meniskusteil mit einer Lagerfläche für diese Kondyle aufweisen.

Vorteilhafterweise ist die jeweilige Kondyle des ersten Prothesenteils konvex und die zugehörige Lagerfläche des Meniskusteils entsprechend konkav. Dies entspricht dem natürlichen Gelenkaufbau. Es wäre aber auch möglich, wo die anatomische Struktur dies nahelegt, die jeweilige Kondyle des ersten Prothesenteils als konkave Lagerfläche und die entsprechende Lagerfläche des Meniskusteils als konvexe Kondyle zu gestalten.

Bei der Ausbildung der Prothese als Kniegelenkprothese ist es vorteilhaft, wenn das zweite Prothesenteil, das Zwischenteil und gegebenenfalls das gemeinsame Meniskusteil eine Ausnehmung für das hintere Kreuzband aufweisen. Die erfindungsgemässe Ausbildung der Prothese lässt solche Ausnehmungen zu. Dies hat den Vorteil, dass die für den Bewegungsablauf des Kniegelenks wichtige Funktion des hinteren Kreuzbandes aufrechterhalten wird.

Ausführungsbeispiele der Erfindung werden nun unter Bezugnahme auf die Zeichnung beschrieben. Es zeigt:
- Fig.1: die Kraftumsetzung in Translationsbewegung bei einem Schnittgelenk,
- Fig.2: die Scherkraftbildung am Interface bei einem Scharniergelenk,
- Fig. 3: die Verhältnisse bei einem "geöffneten" Scharniergelenk,
- Fig. 4: die Verhältnisse bei einem kombinierten Schlitten- und Scharniergelenk,
- Fig. 5a: ein erstes Ausführungsbeispiel einer Kniegelenkprothese im Schnitt, wobei das erste Prothesenteil durch eine gestrichelte Linie gezeugt ist,
- Fig. 5b: eine Explosionsdarstellung der Prothese von Fig. 5a,
- Fig. 6: eine Ansicht von oben auf die Kniegelenkprothese gemäss Fig. 5a,
- Fig. 7: einen Schnitt durch ein zweites Ausführungsbeispiel einer Kniegelenkprothese,
- Fig. 8: eine Ansicht von oben auf die Kniegelenkprothese von Fig. 7,
- Fig. 9a: einen Schnitt durch ein drittes Ausführungsbeispiel einer Kniegelenkprothese,
- Fig. 9b: eine Explosionsdarstellung der Prothese von Fig. 9a,
- Fig. 10: eine Ansicht von oben auf die Kniegelenkprothese von Fig. 9a,
- Fig. 11a: einen Schnitt durch ein viertes Ausführungsbeispiel einer Kniegelenkprothese,
- Fig. 11b: eine Explosionsdarstellung der Prothese von Fig. 11a und
- Fig. 12: eine Ansicht von oben auf die Kniegelenkprothese von Fig. 11a.

Bei den bereits einleitend behandelten verschiedenen bekannten Gelenkformen der Figuren 1 bis 4 ist die Tibia mit der Bezugsziffer 1 bezeichnet. Auf der Tibia 1 ist das untere Gelenkteil 17 befestigt. Die Bezugsziffer 11 bezeichnet das obere Gelenkteil. In Fig. 4 findet sich noch ein auf dem unteren Gelenkteil 17 verschiebbares Meniskusteil 13.

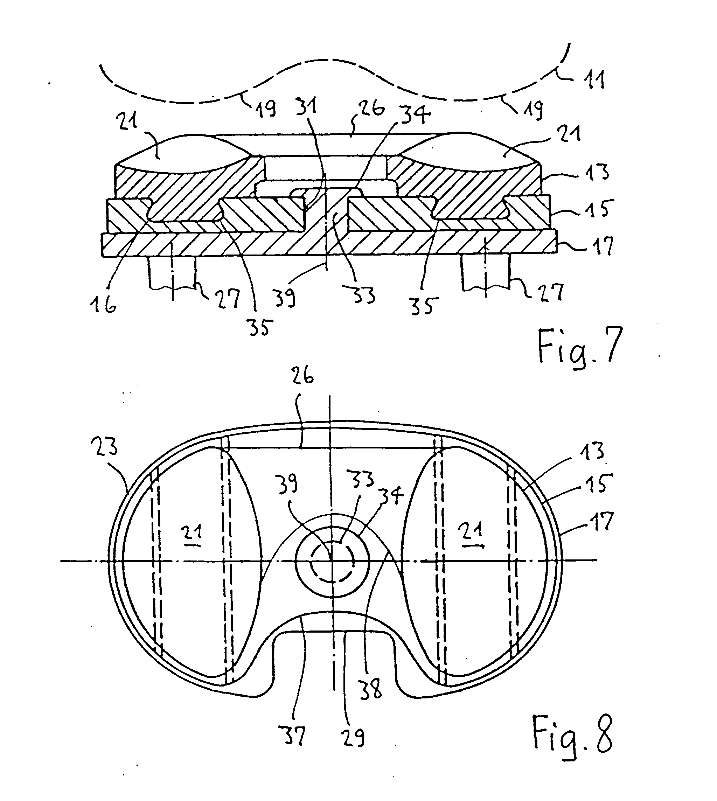

Die in den Figuren 5a, 5b und 6 dargestellte bikompartimentäre Kniegelenkprothese besitzt ein erstes Prothesenteil 11, ein Zwischenteil 15 mit zwei Meniskusteilen 13 und ein zweites Prothesenteil 17. Jedes Meniskusteil 13 besitzt ein Führungsorgan 16, das in einer Führungsbahn 35 im Zwischenteil 15 laufen kann.

Das erste Prothesenteil 11 kann in herkömmlicher Weise mindestens einen Verankerungsabschnitt und mindestens einen Drehgelenkabschnitt 19 aufweisen. Für die vorliegende Erfindung ist natürlich von Bedeutung, dass der Drehgelenkabschnitt 19 des ersten Prothesenteils 11 mit jenem des Meniskusteils 13 übereinstimmt. Wie in Figuren 5a, 5b dargestellt, wird der Drehgelenkabschnitt des ersten Prothesenteils 11 durch zwei Kondylen 19 gebildet. Diesen Kondylen 19 entsprechen die Lagerflächen 21 der Meniskusteile 13.

Das zweite Prothesenteil 17 besteht aus einer eine Gleitfläche 24 aufweisenden Platte 23, von welcher sich konische Verankerungsabschnitte 27 nach unten erstrecken. In Fig. 5b wird gezeigt, dass die Oberseite der Platte 23 eine Schicht 23' aus Kunststoff aufweisen kann. Wie Fig. 6 zeigt, besitzt die Platte 23 eine den Umrissen der natürlichen Tibiakondylen angepasste ovale Formgebung mit einer posterioren Ausnehmung 29 für das hintere Kreuzband. Das zweite Prothesenteil 17 weist einen Drehzapfen 33 auf, welcher in eine Bohrung 31 des Zwischenteils 15 passt. Eine Ueberdachung 34 verhindert ein Abheben des Zwischenteils 15 von der Gleitfläche 24. Es kann auch z.B. eine Schraube 34' (Fig. 5b) oder dergleichen vorgesehen sein. Im Zwischenteil 15 befinden sich zwei Führungsbahnen 35 in Form von Schwalbenschwanz-Nuten. Sie könnten aber auch einen anderen geeigneten Querschnitt aufweisen. Die Führungsbahnen sind praktisch parallel zueinander im Zwischenteil 15 angeordnet und verlaufen ungefähr sagittal.

Wie Fig. 6 zeigt, hat das Zwischenteil 15 eine ähnliche Formgebung wie die Platte 23, ist aber in sagittaler Richtung schmäler als die Platte 23. Das Zwischenteil 15 weist ebenfalls eine Ausnehmung 37 für das hintere Kreuzband auf.

Bei einer Betrachtung der Figuren 5a und 6 kann man erkennen, dass das Zwischenteil 15 eine Schwenkbewegung um die Achse 39 und die Meniskusteile 13 eine Translationsbewegung von anterior nach posterior und umgekehrt durchführen können. Diese Bewegungen werden nach dem Einbau der Prothese durch die Bänder des Kniegelenks geführt und begrenzt. Der Bewegungsablauf bei Flexion und Extension entspricht weitgehend dem physiologischen Bewegungsablauf des Kniegelenks.

Das erste und das zweite Prothesenteil 11, 17 sowie das Zwischenteil 15 bestehen vorteilhaft aus einer Metallegierung, wie sie üblicherweise für Gelenkprothesen Anwendung findet. Die Meniskusteile 13 bestehen zweckmässigerweise aus einem Kunststoff, wie er ebenfalls üblicherweise für Gelenkprothesen verwendet wird. Es ist jedoch auch möglich, wegen der hohen Kongruenz der Lagerflächen 21 und der Kondylen 19, die Meniskusteile 13 ebenfalls aus Metall zu fertigen. Andererseits kann jedoch auch, wo dies opportun erscheint, eine der beiden zueinander gewandten Gleitflächen 24, 28 des zweiten Prothesenteils 17, bzw. des zweiten Zwischenteils 15 mit Kunststoff belegt werden. Es ist also möglich, immer eine Paarung von Kunststoff und Metall bei den verschiedenen Gleitflächen vorzusehen oder sämtliche Gleitflächen in Metall- oder anderer zweckmässiger Hartstoffpaarung auszuführen.

Beim gezeigten Ausführungsbeispiel kommen zwei Kondylen/Lagerflächen-Paare 19, 21 zur Anwendung. Es ist aber auch möglich, nur ein Kondylen/Lagerflächen-Paar zu verwenden.

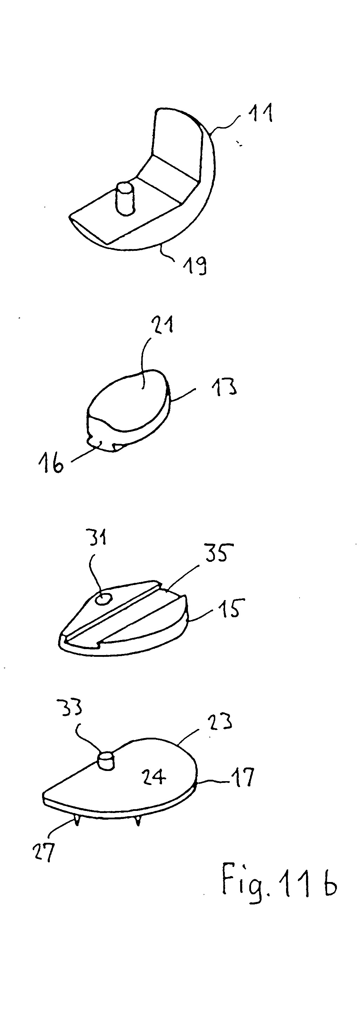

Die Ausführungsform gemäss den Figuren 7 und 8 unterscheidet sich von jener von Fig. 13a, 13b und 14 dadurch, dass für beide Kondylen 19 ein gemeinsames Meniskusteil 13 vorgesehen ist, welches eine Ausnehmung 38 für das hintere Kreuzband aufweist. Man kann sich also die beiden Meniskusteile 13 der Figuren 13a, 13b und 14 durch eine Brücke 26 (Figur 7) verbunden vorstellen. Zweckmässigerweise ist aber das gemeinsame Meniskusteil 13 von Figur 7 einstückig. Er weist zwei Lagerflächen 21 auf. Im übrigen ist die Prothese gleich ausgebildet wie in Figuren 5a, 5b und 6, so dass für Einzelheiten auf die vorherige Beschreibung verwiesen werden kann.

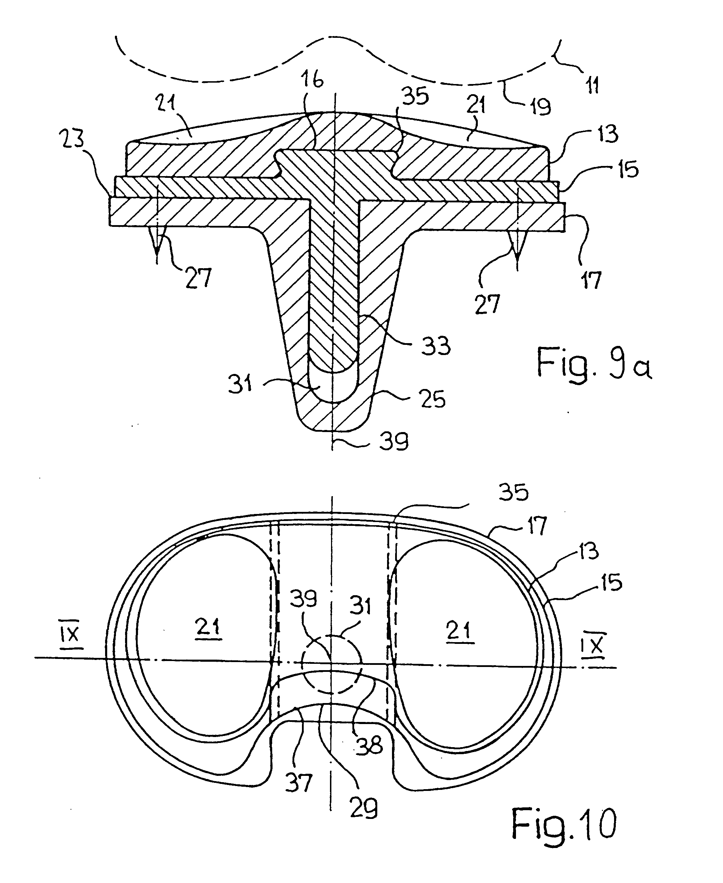

Auch bei der Ausführungsform gemäss den Figuren 9a, 9b und 10 ist für beide Kondylen 19 ein gemeinsames Meniskusteil 13 vorgesehen. Ein Unterschied zu den vorher beschriebenen Ausführungsbeispielen besteht aber darin, dass nicht das zweite Prothesenteil 17, sondern das Zwischenteil 15 einen Drehzapfen 33 aufweist, welcher in einer Bohrung 31 des relativ grossen Verankerungsabschnitts 25 gelagert ist. Die konischen Verankerungsabschnitte 27 sind relativ klein ausgebildet. Ein weiterer Unterschied besteht darin, dass die Führungsbahn 35 des Zwischenteils 15 als Steg ausgebildet ist und das Führungsteil 16 des Meniskusteils 13 als eine den Steg umfassende Nut. Auch das Meniskusteil 13 weist dorsal eine Ausnehmung 38 für das hintere Kreuzband auf. In Fig. 9b wird gezeigt, dass die Unterseite des zweiten Zwischenteils 15 eine Schicht 32 aus Kunststoff aufweisen kann. Im übrigen wird wiederum auf die Beschreibung in bezug auf die Figuren 5a, 5b und 6 verwiesen.

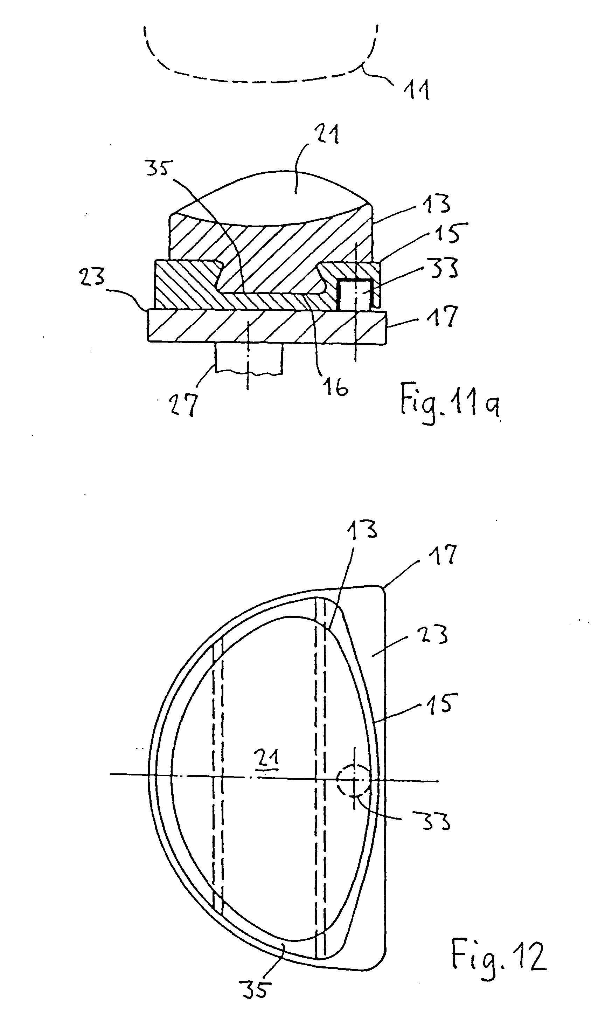

In den Figuren 11a, 11b und 12 ist eine monokompartimentäre Gelenkprothese dargestellt, welche grundsätzlich den gleichen Aufbau aufweist wie die bikompartimentäre Gelenkprothese gemäss den Figuren 5a, 5b und 6. Es kann daher auch auf die vorangehende Beschreibung verwiesen werden. Wenn in Fig. 12 keine Mittel dargestellt sind, welche analog zur Ueberdachung 34 (Fig. 5a) ein Abheben des Zwischenteils 33 verhindern, so ist doch dem Fachmann klar, dass durch geeignete Massnahmen, z.B. durch Anbringen eines Seegerringes, ein Abheben verhindert werden kann.

## Patentansprüche

1. Gelenkprothese, insbesondere Kniegelenkprothese, mit
mindestens einem ersten Prothesenteil (11), das einen Verankerungsabschnitt und einen Drehgelenkabschnitt (19) aufweist,
einem zweiten Prothesenteil (17), das eine Gleitfläche (24) aufweisende Platte (23) und einen Verankerungsabschnitt (25,27) aufweist,
mindestens einem Meniskusteil (13) mit mindestens einem Drehgelenkabschnitt (21) zur Zusammenarbeit mit dem entsprechenden Drehgelenkabschnitt (19) des ersten Prothesenteils (11) und mit einem Führungsorgan (16),
**gekennzeichnet durch**
ein Zwischenteil (15), das eine ähnliche Formgebung wie die Platte (23) des zweiten Prothesenteils (17) aufweist, und auf weichem das Meniskusteil (16) angeordnet ist und gleiten kann,
wobei das Zwischenteil (15) eine Führungsbahn (35) für das Führungsorgan (16) aufweist, durch welche das Meniskusteil (13) in angenähert physiologischer Gleitrichtung geführt wird, um eine Translationsbewegung zu ermöglichen, und auf dem zweiten Prothesenteil (17) verschwenkbar gelagert ist, um dem Meniskusteil (13) zusätzlich zur Translationsbewegung auch eine Schwenkbewegung relativ zum zweiten Prothesenteil (17) zu ermöglichen.

2. Gelenkprothese nach Anspruch 1, **dadurch gekennzeichnet, dass** die jeweilige Führungsbahn (35) geradlinig verläuft.

3. Gelenkprothese nach Anspruch 1 oder 2, **dadurch gekennzeichnet, dass** am zweiten Prothesenteil (17) ein Drehzapfen (33) ausgebildet ist, um welchen das Zwischenteil (15) verschwenkbar ist.

4. Gelenkprothese nach Anspruch 1 oder 2, **dadurch gekennzeichnet, dass** das Zwischenteil (15) einen Drehzapfen (33) aufweist, welcher im zweiten Prothesenteil (17) verschwenkbar gelagert ist.

5. Gelenkprothese nach einem der Ansprüche 1 bis 4, **dadurch gekennzeichnet, dass** das zweite Prothesenteil (17) eine Gleitfläche (24) aufweist, auf welcher das Zwischenteil (15) gelagert ist.

6. Gelenkprothese nach einem der Ansprüche 1 bis 5, **dadurch gekennzeichnet, dass** Mittel vorgesehen sind, um ein Abheben des Zwischenteils (15) vom zweiten Prothesenteil (17) zu verhindern.

7. Gelenkprothese nach einem der Ansprüche 1 bis 6, **dadurch gekennzeichnet, dass** die Beweglichkeit des Meniskusteils (13) relativ zum Zwischenteil (15) auf Bewegungen entlang der Führungsbahn (35) eingeschränkt ist.

8. Gelenkprothese nach einem der Ansprüche 1 bis 7, **dadurch gekennzeichnet, dass** die Führungsbahn (35) und Führungsorgan (15) als Nut und Steg, als Schwalbenschwanz, als Ueberdachung oder bogenförmige Umfassung ausgebildet sind.

9. Gelenkprothese nach einem der Ansprüche 1 bis 8, **dadurch gekennzeichnet, dass** der Drehgelenkabschnitt des ersten Prothesenteils (11) durch zwei Kondylen (19) gebildet ist und für jede dieser Kondylen (19) ein Meniskusteil (13) mit einer Lagerfläche (21) für die Kondylen (19) vorgesehen ist.

10. Gelenkprothese nach einem der Ansprüche 1 bis 8, **dadurch gekennzeichnet, dass** der Drehgelenkabschnitt des ersten Prothesenteils (11) durch zwei Kondylen (19) gebildet ist und dass ein für beide Kondylen (19) gemeinsames Meniskusteil (13) mit einer Lagerfläche (21) für jede der Kondylen (19) vorgesehen ist.

11. Gelenkprothese nach Anspruch 10, **dadurch gekennzeichnet, dass** das gemeinsame Meniskusteil (13) durch zwei in einem Abstand voneinander angeordnete Führungsbahnen (35) geführt wird.

12. Gelenkprothese nach einem der Ansprüche 1 bis 11, **dadurch gekennzeichnet, dass** das zweite Prothesenteil (17) und das Zwischenteil (15) eine Ausnehmung (29, 37) für das Kreuzband aufweist.

13. Gelenkprothese nach Anspruch 10 oder 11, **dadurch gekennzeichnet, dass** das für beide Kondylen (19) gemeinsame Meniskusteil (13) eine Ausnehmung (38) für das Kreuzband aufweist.

14. Gelenkprothese nach einem der Ansprüche 1 bis 8, **dadurch gekennzeichnet, dass** der Drehgelenkabschnitt des ersten Prothesenteils (11) durch eine Kondyle (19) gebildet ist und dass das Zwischenteil (15) ein Meniskusteil (13) mit einer Lagerfläche (21) für diese Kondyle (19) aufweist.

15. Gelenkprothese nach einem der Ansprüche 1 bis 14, **dadurch gekennzeichnet, dass** die jeweilige Kondyle (19) des ersten Prothesenteils (11) konvex, die zugehörige Lagerfläche (21) des Meniskusteils (13) entsprechend konkav ist.

16. Gelenkprothese nach einem der Ansprüche 1 bis 15, **dadurch gekennzeichnet, dass** die jeweilige Kondyle (19) des ersten Prothesenteils (11) konkav und zugehörige Lagerfläche (21) des Meniskusteils (13) konvex ist.

17. Gelenkprothese nach einem der Ansprüche 1 bis 15, **dadurch gekennzeichnet, dass** bei den Gleitflächen eine Paarung von Kunststoff und Metall vorgesehen ist.

18. Gelenkprothese nach einem der Ansprüche 1 bis 15, **dadurch gekennzeichnet, dass** bei den Gleitflächen eine Metall- oder andere Hartstoffpaarung vorgesehen ist.

## Claims

1. A joint prosthesis, more particularly a knee-joint prosthesis, comprising
at least one first prosthesis part (11) comprising an anchoring portion and at least one rotary joint portion (19),
a second prosthesis part (17) comprising at least one plate (23) with a slide surface (24) and an anchoring portion (25,27),
a meniscus part (13) comprising at least one rotary joint portion (21) for cooperating with the corresponding rotary joint portion (19) of the first prosthesis part (11) and a guiding means (16),
**characterised by** an intermediate part (15) having a similar form as the plate (23) of the second prosthesis part (17) and on which the meniscus part (13) is located and can slide thereon,
the intermediate part (15) having a guide track (35) for the guiding means (16) by which the meniscus part (13) is guided in substantially physiological sliding direction to permit a translatory movement, and being located pivotally movable on the second prosthesis part (17) to permit, in addition to the translatory movement, also a pivotal movement with respect to the second prosthesis part (17).

2. A joint prosthesis according to claim 1, **characterised in that** the guide track (35) extends in a straight line.

3. A joint prosthesis according to claim 1, **characterised in that** on the second prosthesis part (17) a pivot (33) is disposed, on which the intermediate part (15) can pivot.

4. A joint prosthesis according to claim 1 or 2, **characterised in that** the intermediate part (15) has a pivot (33) which is pivotally movable in the second prosthesis part (17).

5. A joint prosthesis according to any of the claims 1 to 4, **characterised in that** the second prosthesis part (17) comprises a slide surface (24) on which the intermediate part (15) is located.

6. A joint prosthesis according to any of claims 1 to 5, **characterised in that** means are provided for preventing the intermediate part (15) from being lifted off the second prosthesis part (17).

7. A joint prosthesis according to any of the claims 1 to 6, **characterised in that** the mobility of the meniscus part (13) relative to the intermediate (15) is restricted to motion along the guide track (35).

8. A joint prosthesis according to any of the claims 1 to 7, **characterised in that** the guide track (35) and the guide part (15) are in the form of a groove and web, a dovetail, a roof or an arcuate enveloping part.

9. A joint prosthesis according to any of the claims 1 to 8, **characterised in that** the rotary joint portion of the first prosthesis part is formed by two condyles (19) and that for each of said condyles (19) a meniscus part (13) with a bearing surface (21) for the condyles (19) is provided.

10. A joint prosthesis according to any of claims 1 to 8, **characterised in that** the rotary joint portion of the first prosthesis part (11) is formed by two condyles (19) and that a meniscus part (13) having a bearing surface (21) common to both condyles (19) is provided for each of the condyles (19).

11. A joint prosthesis according to claim 10, **characterised in that** the common meniscus part (13) is guided by two spaced-apart guide tracks (35).

12. A joint prosthesis according to any of claims 1 to 11, **characterised in that** the second prosthesis part (17) and the intermediate part (15) have a recess (29,37) for the cruciate ligament.

13. A joint prosthesis according to claim 10 or 11, **characterised in that** the meniscus part (13) common to both condyles (19) has a recess (38) for the cruciate ligament.

14. A joint prosthesis according to any of claims 1 to 8, **characterised in that** the rotary joint portion of the first prosthesis part (11) is formed by a condyle (19) and that the intermediate part (15) has a meniscus part (13) with a bearing surface (21) for this condyle (19).

15. A joint prosthesis according to any of claims 1 to 14, **characterised in that** the condyle (19) on the first prosthesis part (11) is convex and the associated bearing surface (21) on the meniscus part (13) is correspondingly concave.

16. A joint prosthesis according to any of claims 1 to 15, **characterised in that** the condyle (19) on the first prosthesis part (11) is concave and the corresponding bearing surface (21) on the meniscus part (13) is correspondingly convex.

17. A prosthesis according to any of the claims 1 to 15, **characterised in that** at the slide surfaces a pairing of plastics and metal is provided.

18. A prosthesis according to any of the claims 1 to 15, **characterised in that** at the slide surfaces metal or a hard material pairing is provided.

## Revendications

1. Prothèse d'articulation, notamment une prothèse d'articulation de genou, comprenant
au moins un premier élément de prothèse (11) présentant une section d'ancrage et une section d'articulation à charnière (19),
un deuxième élément de prothèse (17) présentant un plateau (23) comprenant une surface de glissement (24) et une section d'ancrage (25, 27),
au moins un élément de ménisque (13) comprenant au moins une section d'articulation à charnière (21) pour coopérer avec la section d'articulation à charnière (19) correspondante du premier élément de prothèse (11), et un organe de guidage (16) ,
**caractérisée par** un élément intermédiaire (15) présentant une forme similaire que celle du plateau (23) du deuxième élément de prothèse (17) et sur lequel est disposé et peut glisser l'élément de ménisque (16), l'élément intermédiaire (15) présentant une voie de guidage (35) pour l'organe de guidage (16) par lesquels l'élément de ménisque est guidé dans un sens de glissement approximativement physiologique pour permettre un mouvement de translation, et qui est logé pivotant sur le deuxième élément de prothèse (17) pour permettre à l'élément de ménisque (13), en plus du mouvement de translation, un mouvement de pivotement par rapport au deuxième élément de prothèse (17).

2. Prothèse d'articulation selon la revendication 1, **caractérisée en ce que** la voie de guidage (35) respective est rectiligne.

3. Prothèse d'articulation selon la revendication 1 ou 2, **caractérisée en ce que** sur le deuxième élément de prothèse (17) un tourillon (33) est prévu, autour duquel peut pivoter l'élément intermédiaire (15).

4. Prothèse d'articulation selon la revendication 1 ou 2, **caractérisée en ce que** l'élément intermédiaire (15) présente un tourillon (33) logé pivotant dans le deuxième élément de prothèse (17).

5. Prothèse d'articulation selon l'une des revendications 1 à 4, **caractérisée en ce que** le deuxième élément de prothèse (17) présente une surface de glissement (24) sur laquelle est logé l'élément intermédiaire (15).

6. Prothèse d'articulation selon l'une des revendications 1 à 5, **caractérisée en ce que** des moyens sont prévus pour empêcher un soulèvement de l'élément intermédiaire (15) du deuxième élément de prothèse (17).

7. Prothèse d'articulation selon l'une des revendications 1 à 6, **caractérisée en ce que** la mobilité de l'élément de ménisque (13) par rapport à l'élément intermédiaire (15) est limitée à des mouvements le long de la voie de guidage (35).

8. Prothèse d'articulation selon l'une des revendications 1 à 7, **caractérisée en ce que** la voie de guidage (35) et l'organe de guidage (15) sont configurés comme rainure et nervure, en queue d'aronde, comme couverture ou comme bordure en forme d'arc.

9. Prothèse d'articulation selon l'une des revendications 1 à 8, **caractérisée en ce que** la section d'articulation à charnière du premier élément de prothèse (11) est constituée par deux condyles (19) et que pour chacun de ces condyles (19) un élément de ménisque (13) avec une surface de logement (21) pour les condyles est (19) prévu.

10. Prothèse d'articulation selon l'une des revendications 1 à 8, **caractérisée en ce que** la section d'articulation à charnière du premier élément de prothèse (11) est constituée par deux condyles (19) et qu'un élément de ménisque (13) commun aux deux condyles (19) présentant une surface de logement (21) pour chacun des condyles (19) est prévu.

11. Prothèse d'articulation selon la revendication 10, **caractérisée en ce que** l'élément de ménisque (13) commun est guidé par deux voies de guidage (35) écartées l'une de l'autre.

12. Prothèse d'articulation selon l'une des revendications 1 à 11, **caractérisée en ce que** le deuxième élément de prothèse (17) et l'élément intermédiaire (15) présentent une cavité (29, 37) pour le ligament croisé arrière.

13. Prothèse d'articulation selon la revendication 10 ou 11, **caractérisée en ce que** l'élément de ménisque (13) commun des deux condyles (19) présente une cavité (38) pour le ligament croisé.

14. Prothèse d'articulation selon l'une des revendications 1 à 8, **caractérisée en ce que** la section d'articulation à charnière du premier élément de prothèse (11) est constituée par un condyle (19), et que l'élément intermédiaire (15) présente un élément de ménisque (13) avec une surface de logement (21) pour ces condyles (19).

15. Prothèse d'articulation selon l'une des revendications 1 à 14, **caractérisée en ce que** le condyle (19) respectif du premier élément de prothèse (11) est convexe et la surface de logement (21) correspondante de l'élément de ménisque (13) concave.

16. Prothèse d'articulation selon l'une des revendications 1 à 15, **caractérisée en ce que** le condyle (19) respectif du premier élément de prothèse (11) est concave et la surface de logement (21) correspondante de l'élément de ménisque (13) convexe.

17. Prothèse d'articulation selon l'une des revendications 1 à 15, **caractérisée en ce que** pour les surfaces de glissement une combinaison de matière plastique et de métal est prévue.

18. Prothèse d'articulation selon l'une des revendications 1 à 15, **caractérisée en ce que** pour les surfaces de glissement une combinaison de métal ou d'autres matières dures est prévue.
